# EUROPEAN PATENT SPECIFICATION

(11) **EP 3 930 814 B1**
(45) Date of publication and mention of the grant of the patent: **04.11.2026**
(21) Application number: 20799370.0
(22) Date of filing: 30.04.2020
(51) Int. Cl.: A61M 25/00, A61B 1/005, A61M 25/01, A61M 25/09, B26D 3/12, B26D 3/14

(54) **INTRAVASCULAR DEVICE WITH ENHANCED ONE-BEAM CUT PATTERN**
INTRAVASKULÄRE VORRICHTUNG MIT VERBESSERTEM EINSTRAHLSCHNITTMUSTER
DISPOSITIF INTRAVASCULAIRE À MOTIF DE COUPE À UN SEUL FAISCEAU AMÉLIORÉ

(30) Priority: 02.05.2019 US 201962842216 P; 22.04.2020 US 202016855366
(43) Date of publication of application: 05.01.2022
(73) Proprietor: Scientia Vascular, Inc., West Valley City, UT 84119 (US)
(72) Inventor: SNYDER, Edward J., Park City, Utah 84098 (US)
(74) Representative: Hepworth Browne
(86) International application number: PCT/US2020/030589
(87) International publication number: WO 2020/223423

(56) References cited:
- US-A1- 2004 167 437
- US-A1- 2006 189 896
- US-A1- 2006 189 896
- US-A1- 2009 043 372
- US-A1- 2010 145 308
- US-A1- 2010 256 528
- US-A1- 2010 256 528
- US-A1- 2014 336 620
- US-A1- 2017 189 643
- US-A1- 2017 189 643
- US-A1- 2018 071 496
- US-B1- 6 428 489
- US-B1- 8 409 169

## Description

### CROSS-REFERENCE TO RELATED APPLICATIONS

This application claims priority to United States Patent Application Serial No. 16/855,366, filed April 22, 2020 and entitled "INTRAVASCULAR DEVICE WITH ENHANCED ONE-BEAM CUT PATTERN," which application claims priority to and the benefit of United States Provisional Patent Application Serial No. 62/842,216, filed May 2, 2019 and titled "INTRAVASCULAR DEVICE WITH ENHANCED ONE-BEAM CUT PATTERN,".

### BACKGROUND

Interventional devices such as guidewires and catheters are frequently utilized in the medical field to perform delicate procedures deep within the human body. Typically, a catheter is inserted into a patient's femoral, radial, carotid, or jugular vessel and navigated through the patient's vasculature to the heart, brain, or other targeted anatomy as required. Often, a guidewire is first routed to the targeted anatomy, and one or more catheters are subsequently passed over the guidewire and routed to the targeted anatomy. Once in place, the catheter can be used to deliver drugs, stents, embolic devices, radiopaque dyes, or other devices or substances for treating the patient in a desired manner.

In many applications, such an interventional device must be angled through the tortuous bends and curves of a vasculature passageway to arrive at the targeted anatomy. For example, directing a guidewire and/or catheter to portions of the neurovasculature requires passage through the internal carotid artery and other tortuous paths. Such an interventional device requires sufficient flexibility, particularly closer to its distal end, to navigate such tortuous pathways.

In some cases, portions of the device are microfabricated to increase flexibility. For example, a guidewire may include an outer elongated tube that includes a series of machine-cut fenestrations near the distal end and sometimes at other locations. The cuts are typically arranged to define a series of axially extending "beams" that connect a series of circumferentially extending "rings."

While such microfabricating techniques are beneficial for increasing the flexibility of elongated intravascular components, several challenges remain. There is thus a long felt and ongoing need for improved intravascular devices and methods that enable the manufacture of such devices.

US 2004/167437 discloses intracorporal medical devices and a method of making and using the same. An intracorporal medical device includes a proximal section and a distal section. An articulating member may be disposed adjacent the proximal and distal sections. The articulating member may provide the intracorporal medical device with improved bending characteristics.

US 2010/145308 discloses medical devices and methods for making and using the same. A medical device may include an elongate tubular member. The tubular member may include a first circumferential tube segment, a second circumferential tube segment disposed next to the first circumferential tube segment, and a third circumferential tube segment disposed next to the second circumferential tube segment. The first tube segment and the second tube segment may be separated by a first set of slots formed in the tubular member. The second tube segment and the third tube segment may be separated by a second set of slots formed in the tubular member. The second tube segment may be connected to the first tube segment with a proximally-extending beam formed in the tubular member. The second tube segment may also be connected to the third tube segment with a distally-extending beam formed in the tubular member. A ring may be defined in the second tube segment between the proximally-extending beam and the distally-extending beam. The ring may have a first portion with a first width and a second portion with a second width different from the first width.

See further, e.g., US2018071496 A1 and US 8409169 B1.

### SUMMARY

Disclosed are intravascular devices, including guidewires and microcatheters, having enhanced one-beam cut patterns. An elongated member includes a plurality of fenestrations that define a plurality of axially extending beams interspersed between a plurality of circumferentially extending rings. The beams are formed using a dual-pass cutting method in which a blade makes two rotationally offset cutting passes at each longitudinal location of the elongated member. The resulting beam has enhanced structural features that avoid overly sharp edges and minimize structural weak points.

The invention provides, an intravascular device according to claim 1.

The invention provides, a method of manufacturing an intravascular device according to claim 11.

It is to be understood that both the foregoing general description and the following detailed description are exemplary and explanatory only and are intended to provide further explanation of the invention as claimed.

### BRIEF DESCRIPTION OF THE DRAWINGS

Various objects, features, characteristics and advantages of the invention will become apparent and more readily appreciated from the following description of the embodiments, taken in conjunction with the accompanying drawings and the appended claims, all of which form a part of this specification wherein like reference numerals designate corresponding parts in the various figures and wherein the various elements depicted are not necessarily drawn to scale; and wherein:
Figure 1 illustrates an exemplary intravascular device such as a guidewire or microcatheter;
Figure 2 illustrates the distal end of an embodiment of an intravascular device configured as a guidewire;
Figure 3 illustrates an elongated member with a one-beam cut pattern;
Figure 4 illustrates a typical process for forming a one-beam cut pattern in a piece of stock material;
Figures 5A and 5B illustrate the structure of a beam that results from the standard cutting procedure shown in Figure 4; and
Figures 6A through 6C illustrate an alternative cutting procedure and the improved structure of a beam that results from the alternative cutting procedure.

In the following, 1 inch can be converted into 25.4 mm.

### DETAILED DESCRIPTION

### Overview of Intravascular Devices

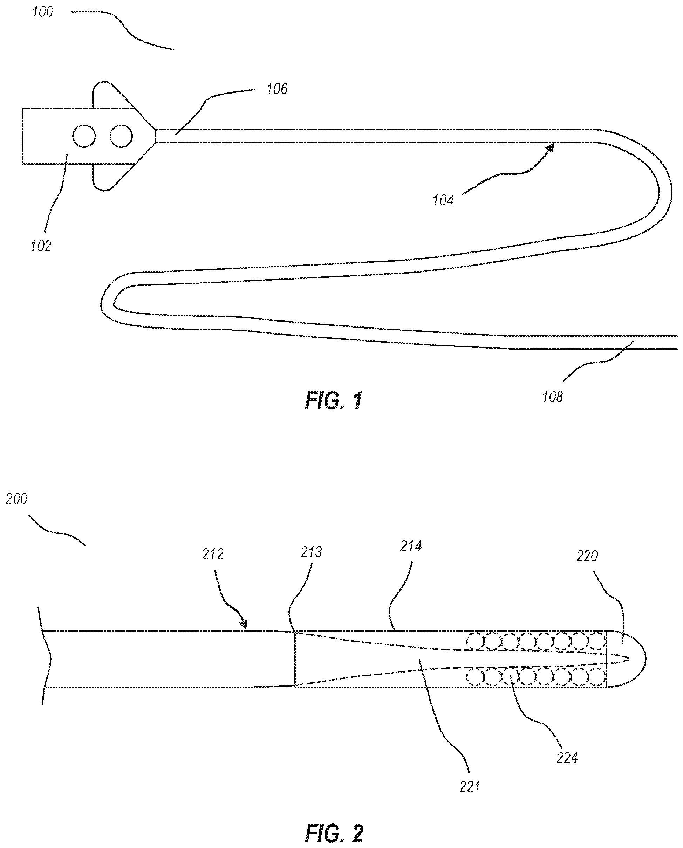

Figure 1 illustrates an exemplary intravascular device 100 that comprises an elongated member 104 extending between a proximal end 106 and a distal end 108. An optional handle/hub/torquer 102 may be attached at the proximal end 106. The elongated member 104 may be, for example, a guidewire or a microcatheter.

The elongated member 104 may include a plurality of fenestrations cut into its outer surface. The fenestrations may be formed by cutting one or more pieces of stock material to form a cut pattern which leaves the fenestrations. The fenestrations can provide a variety of benefits, including increasing the flexibility of the elongated member 104. In some embodiments, the fenestrations are arranged to provide enhanced flexibility (relative to a similar section of stock material lacking fenestrations) while maintaining sufficient outer circumferential structure for effectively transmitting torque.

The elongated member 104 may be any length necessary for navigating a patient's anatomy to reach a targeted anatomical area. A typical length may be within a range of about 50 to 300 cm, for example. In a catheter embodiment, the outer diameter of the elongated member 104 may be within a range of about 0.010 inches to about 0.150 inches, though larger or smaller diameters may also be utilized according to preferences and/or application needs. In a guidewire embodiment, the outer diameter of the elongated member 104 may be about 0.014 inches, or may be within a range of about 0.008 to 0.145 inches, though larger or smaller sizes may also be utilized according to user preferences and/or application needs.

The elongated member 104, in a catheter embodiment, is typically formed from a material having an elastic modulus of about 3000 MPa to about 4500 MPa, or about 3500 MPa to about 4000 MPa. In one exemplary embodiment, the elongated member 104 is formed from or includes polyether ether ketone (PEEK). Other polymers with higher moduli may also be utilized where cost and/or fabrication considerations warrant it. In some embodiments, the elongated member 104 includes or is formed from a nickel-titanium alloy having super elastic properties at body temperature. In some embodiments, at least a portion of the elongated member 104 (e.g., a proximal portion) is formed from a stainless steel or other material with similar stress-strain and elastic modulus properties. Typically, if the elongated member 104 is formed from two or more different materials, the higher modulus material(s) are used at more proximal sections and the lower modulus material(s) are used at more distal sections.

Figure 2 illustrates the distal end of an embodiment of an intravascular device configured as a guidewire 200. The embodiment illustrated in Figure 2 may represent the distal end 108 of a guidewire embodiment of the elongated member 104 of Figure 1. The embodiment shown in Figure 2 includes an inner member configured as a core 212. Other embodiments may additionally or alternatively include one or more other inner members, such as one or more inner tube structures.

The illustrated guidewire 200 includes a core 212 and a tube structure 214 coupled to the core 212. As shown, a distal section 221 of the core 212 extends into the tube 214 and is surrounded by the tube 214. In some embodiments, the distal section 221 of the core 212 is ground so as to progressively taper to a smaller diameter (e.g., about 0.002 inches) at the distal end. The distal section 221 of the core 212 may have a round cross-section, rectangular cross-section, or other suitable cross-sectional shape. In this example, the core 212 and the tube 214 have substantially similar outer diameters at the attachment point 213 where they adjoin and attach to one another.

The tube 214 may be coupled to the core 212 (e.g., using adhesive, soldering, and/or welding) in a manner that allows torsional forces to be transmitted from the core 212 to the tube 214 and thereby to be further transmitted distally by the tube 214. A medical grade adhesive 220 may be used to couple the tube 214 to the core 212 at the distal end of the device and to form an atraumatic covering.

The guidewire 200 may also include one or more coils such as coil 224 disposed within the tube 214 so as to be positioned between an outer surface of the distal section of the core 212 and an inner surface of the tube 214. The coil 224 may be formed from a radiopaque material, such as platinum. The illustrated coil 224 is formed as one integral piece. In alternative embodiments, the coil 224 includes a plurality of separate sections stacked, positioned adjacent to one another, and/or interlocked through intertwining.

The tube 214 may include micro-fabricated fenestrations configured to provide effective flexibility and torquability of the intravascular device. Some embodiments may additionally or alternatively include cuts formed in the core 212 itself, such as along the distal section 221 of the core.

### Standard One-Beam Cut Patterns

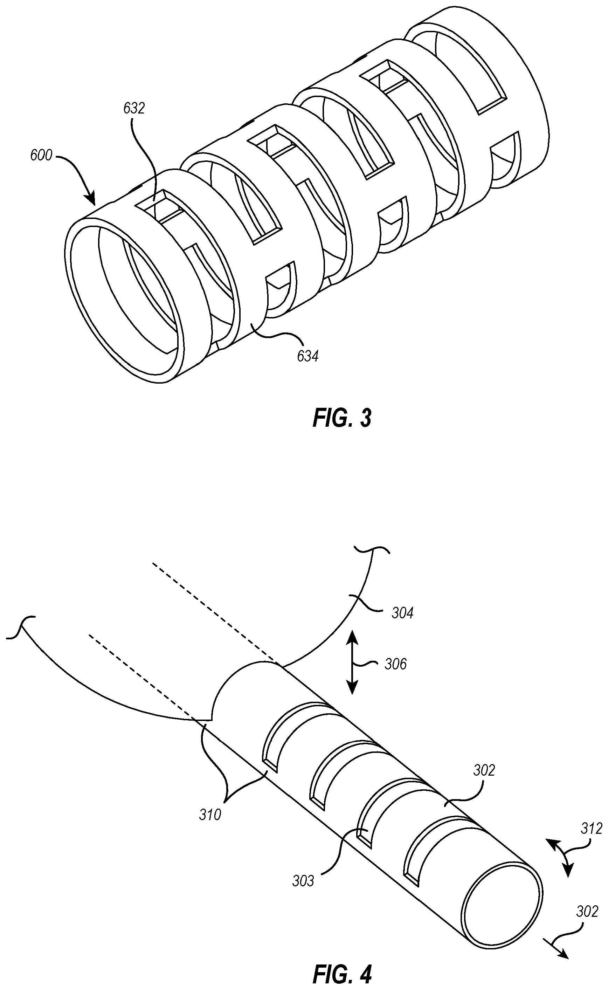

Figure 3 illustrates an elongated member 900 having a plurality of beams 932 and rings 934. The elongated member 900 is an example of a one-beam cut pattern because a single beam 932 is disposed between each pair of adjacent rings 934. In this example, each successive beam is rotationally offset by about 180 degrees from the preceding beam 932. Other embodiments may have different rotational offset patterns or may omit a rotational offset such that the beams are aligned on a single side of the elongated member 900.

Some embodiments may include beams 932 arranged in a "helical" pattern, or a non-linear pattern such as a "distributed" pattern, an "imperfect ramp" pattern, a "sawtooth" pattern, or a combination thereof each in a different section of the elongated member 900. These cut patterns are described in greater detail in PCT International Application Number PCT/US2018/034756, published as International Publication Number WO 2018/218216 A1

In some embodiments, the rings and beams of an intravascular device may be encapsulated in a polymer. Intravascular devices, including guidewires and microcatheters, having polymer encapsulated microfabricated structures are described in greater detail in United States Issued Patent Numbers 9067332, 9950137, 9067333, and 9072873.

Figure 4 illustrates a typical process for forming a one-beam cut pattern in a piece of stock material 302. The stock material 302 (typically a tube structure) is positioned in a cutting machine having a blade 304 (or a plurality of blades). As indicated by arrows 306, the blade 304 is moveable along an axis that is perpendicular to the longitudinal axis of the stock material 302 to form the fenestrations 303. Although the blade 304 is shown here as moving up and down along a vertical axis, other configurations may have a blade (or a plurality of blades) that move along a horizontal axis or even a diagonal axis.

To make a cut, the blade 304 is brought into contact with the stock material 302 and moved inward until the cut is made at the desired depth and a resulting beam 310 remains in the stock material 302. The blade 304 is then withdrawn from the stock material 302. The stock material 302 is then longitudinally moved relative to the blade 304, as indicated by arrow 308, until the next desired cut location is aligned with the blade 304. The process may then be repeated to form the desired number of cuts.

Cut depth and/or spacing between cuts may be varied from one device to the next, or even from one section of a device to another section of the same device. For example, sections intended to form distal portions of an intravascular device may include cuts that are relatively deeper and/or with relatively less spacing in order to increase the relative flexibility at the distal portion.

In some implementations, such as those that form helical or non-linear patterns, the stock material 302 may be rotated between successive cuts or between successive sets of cuts to allow for rotational offsets in the resulting beams, as indicated by arrows 312. Additional details related to cutting machines and related methods of manufacture are described in United States Issued Patent Number 10,232,141.

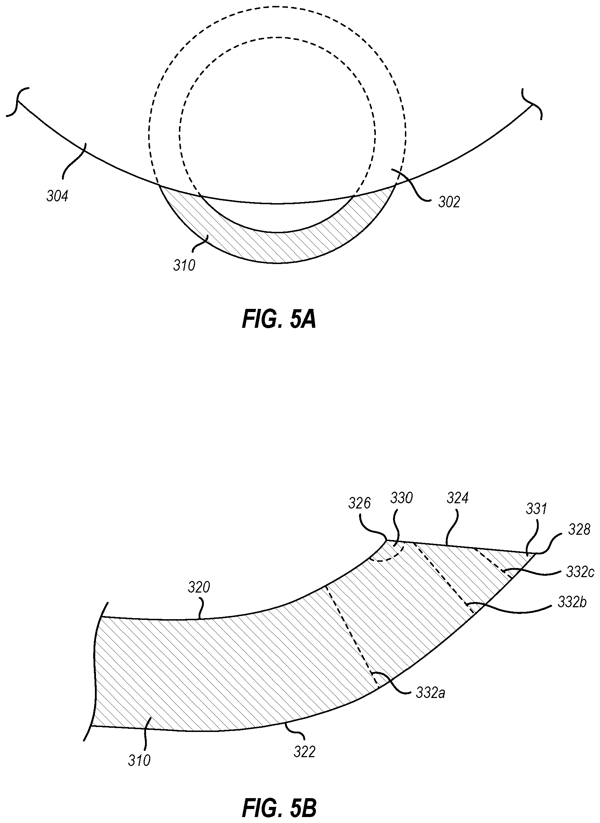

Figures 5A and 5B illustrate in greater detail the structure of the beam 310 that results from the standard cutting procedure shown in Figure 4. Figure 5A shows a front, cross-sectional view of the stock material 302 along a line that runs parallel to the blade path of a particular cut, and Figure 5B shows an expanded view of an edge section of the resulting beam 310. As shown, the blade 304 typically has a diameter significantly larger than the diameter of the stock material 302 (typical blade diameters may range from 2 to 4 inches, for example). Figure 5A shows the blade 304 at its deepest point within the stock material 302. After the blade 304 is withdrawn, the resulting beam 310 remains.

As best shown in Figure 5B, the resulting beam 310 includes an interior surface 320, an exterior surface 322, and two lateral surfaces 324 (only one shown in Figure 5B). Each lateral surface 324 joins the interior surface 320 along an interior edge 326, and joins the exterior surface 322 along an exterior edge 328. Angle 330 is formed where the interior surface 320 joins the lateral surface 324.

Because of the geometry of the cut, angle 330 is significantly greater than 90 degrees, and will typically be about 135 degrees. As a structural consequence of the size of angle 330, the radial thickness of the beam 310 thins out from the interior edge 326 to the exterior edge 328. The "radial thickness" refers to the thickness of the beam along a radial line extending from the geometric center of the cross section of the tube structure 302 to the exterior surface 322. Thus, while the beam 310 has substantially uniform radial thickness across much of its circumferential length (as indicated by radial line 332a), the radial thickness tapers between the interior edge 326 and the exterior edge 328 (as indicated by progressively shorter radial lines 332b and 332c).

Another structural consequence of that edge 328 will be relatively "sharp." That is, the angle 331 formed between lateral surface 324 and exterior surface 322 will be relatively small, such as about 45 degrees or less.

### Enhanced One-Beam Cut Patterns

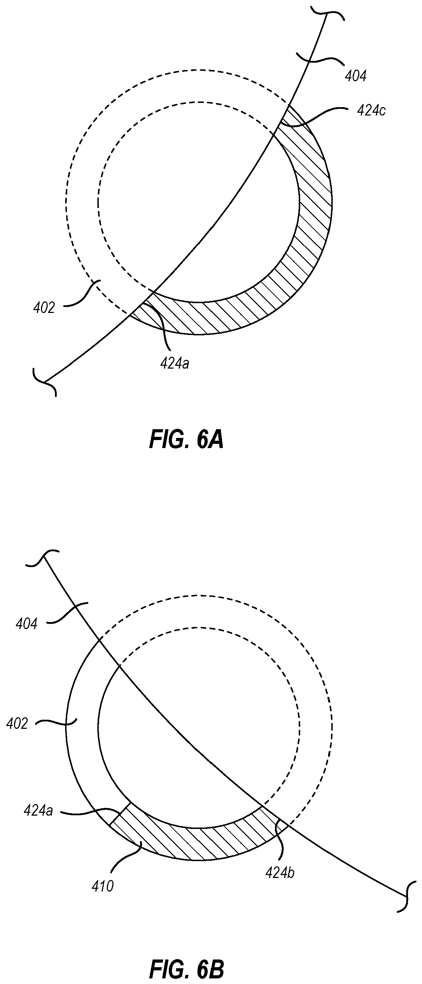

Figures 6A through 6C illustrate an alternative method for forming a beam 410 in a section of stock material 402. As shown in Figure 6A, the blade 404 is first passed into the stock material 402 to a relatively shorter depth compared to the standard cut shown in Figure 5A. For example, where the standard cut shown in Figure 5A typically has a depth of about 70% of the stock material diameter or more, the initial cut depth shown in Figure 6A is approximately 50% (e.g., about 30% to about 70%).

After the initial cut is formed, the stock material 402 is rotated relative to the blade 404 to allow the blade 404 to pass a second time into the stock material 402, as shown in Figure 6B. The stock material 402 maintains the same longitudinal position relative to the blade during the first and second passes of the blade 404 so that the second cut is within the same plane as the first. During the first cut, a first lateral surface 424a is formed and a temporary lateral surface 424c is formed. The second cut then removes the temporary lateral surface 424c and cuts additional material to form the second lateral surface 424b.

Although the sequence from Figure 6A and to Figure 6B gives the appearance that the blade 404 is rotated clockwise relative to the stock material 402, it will be understood that this is for illustrative convenience only, and that any suitable means of relative rotation between the stock material 402 and blade 404 may be utilized by rotating the blade 404, the stock material 402, or both. Typically, the stock material 402 will be rotated relative to a rotationally static blade 404. The relative rotation is preferably about 90 degrees (e.g., about 60 degrees to about 120 degrees, or about 75 degrees to about 105 degrees).

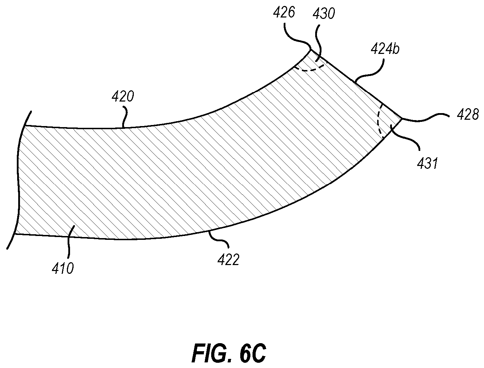

Figure 6C illustrates an expanded view of an edge section of resulting beam 410. The resulting beam 410 includes an interior surface 420, an exterior surface 422, and a pair of lateral surfaces 424 (with the single lateral surface 424b shown here). Each lateral surface 424 joins the interior surface 420 along an interior edge 426, and joins the exterior surface 422 along an exterior edge 428. Angle 430 is formed where the interior surface 420 joins the lateral surface 424.

As compared to angle 330 of the beam 310 shown in Figure 5B, the angle 430 of beam 410 is markedly smaller. For example, the angle 430 may have a value within a range having a lower endpoint of about 75, 80, 85, or 90 degrees and an upper endpoint of about 130, 120, 110, or 100 degrees. Most preferably, the angle 430 is approximately 90 degrees such that lateral surface 424b is substantially perpendicular to interior surface 420.

The structure of the beam 410 provides a marked improvement over the standard beam 310. For example, the beam 410 avoids the "sharp" exterior edge 428 present in the standard beam 310. In other words, the angle 431 formed between the lateral surface 424 and the exterior surface 422 is greater than 45 degrees, such as about 50 degrees to about 90 degrees.

The improved beam 410 also avoids the tapering profile of the standard beam 310, and has more uniform radial thickness across the circumferential length of the beam 410. This beneficially minimizes structural weak points and provides greater fatigue life to the beam 410.

The dual-pass cutting process has also surprisingly been found to increase manufacturing efficiency and yields as compared to the standard, single-pass process. Even though the number of blade passes is doubled, the dual-pass process requires less depth per cut and typically forms more accurate cuts. This has been found to more than make up for the additional time required to do two cuts per beam.

### Conclusion

While certain embodiments of the present disclosure have been described in detail, with reference to specific configurations, parameters, components, elements, etcetera, the descriptions are illustrative and are not to be construed as limiting the scope of the claimed invention.

Furthermore, it should be understood that for any given element of component of a described embodiment, any of the possible alternatives listed for that element or component may generally be used individually or in combination with one another, unless implicitly or explicitly stated otherwise.

In addition, unless otherwise indicated, numbers expressing quantities, constituents, distances, or other measurements used in the specification and claims are to be understood as optionally being modified by the term "about" or its synonyms. When the terms "about," "approximately," "substantially," or the like are used in conjunction with a stated amount, value, or condition, it may be taken to mean an amount, value or condition that deviates by less than 20%, less than 10%, less than 5%, or less than 1% of the stated amount, value, or condition. At the very least, each numerical parameter should be construed in light of the number of reported significant digits and by applying ordinary rounding techniques.

Any headings and subheadings used herein are for organizational purposes only and are not meant to be used to limit the scope of the description or the claims.

It will also be noted that, as used in this specification and the appended claims, the singular forms "a," "an" and "the" do not exclude plural referents unless the context clearly dictates otherwise. Thus, for example, an embodiment referencing a singular referent (e.g., "widget") may also include two or more such referents.

It will also be appreciated that embodiments described herein may include properties, features (e.g., ingredients, components, members, elements, parts, and/or portions) described in other embodiments described herein. Accordingly, the various features of a given embodiment can be combined with and/or incorporated into other embodiments of the present disclosure. Thus, disclosure of certain features relative to a specific embodiment of the present disclosure should not be construed as limiting application or inclusion of said features to the specific embodiment. Rather, it will be appreciated that other embodiments can also include such features.

## Claims

1. An intravascular device (100), comprising:
an elongated member (104) extending between a proximal end (106) and a distal end (108) along a longitudinal axis, and having a plurality of fenestrations that define a plurality of axially extending beams (410) and circumferentially extending rings (634) in a one-beam cut pattern wherein a single axially extending beam (310) is disposed between each pair of adjacent circumferentially extending rings (634);
wherein at least one beam (410) of the one-beam cut pattern includes an interior surface (420), an exterior surface (422), and a pair of lateral surfaces (424) **characterized in that**
an angle (430) formed between the interior surface (420) and one or both of the lateral surfaces (424a, 424b) is between 80 degrees and 120 degrees.

2. The device of claim 1, wherein the elongated member (104) is a tube structure (214).

3. The device of claim 2, further comprising at least one of a core (212) disposed within the tube structure (214), an inner tube disposed within the tube structure, or one or more coils (224) disposed within the tube structure (214).

4. The device of any one of claims 1-3, wherein the rings (634) and beams (410) are encapsulated in a polymer.

5. The device of any one of claims 1-4, wherein the elongated member (104) comprises a nickel-titanium alloy or stainless steel or a polymer.

6. The device of any one of claims 1-5, wherein the elongated member (104) is formed from two or more different materials.

7. The device of any one of claims 1-6, wherein the at least one beam (410) has a substantially uniform thickness across its circumferential length.

8. The device of any one of claims 1-7, wherein an angle (431) formed between the exterior surface (422) and one or both lateral surfaces (424) is greater than about 45 degrees.

9. The device of any one of claims 1-8, wherein the intravascular device (100) is a guidewire.

10. The device of any one of claims 1-8, wherein the intravascular device (100) is a microcatheter.

11. A method of manufacturing an intravascular device (100) according to claim 1, the method comprising:
providing a piece of stock material (402);
passing a blade (404) into the stock material (402) to form a first cut in the stock material (402) without passing completely through the stock material (402), the blade (404) being oriented such that a cutting edge is substantially perpendicular to a longitudinal axis of the stock material (402);
rotating the stock material (402) relative to the blade (404) by about 60 degrees to about 120 degrees without longitudinally advancing the stock material (402) relative to the blade (404); and
passing the blade (404) into the stock material (402) to form a second cut.

12. The method of claim 11, wherein the first cut is made by passing the blade (404) into the stock material (402) to a depth of about 30% to about 70% of the diameter of the stock material (402).

13. The method of claim 11 or claim 12, wherein the second cut is made by passing the blade (404) into the stock material (402) to a depth of about 30% to about 70% of the diameter of the stock material (402).

## Patentansprüche

1. Intravaskuläre Vorrichtung (100), umfassend:
ein längliches Element (104), das sich zwischen einem proximalen Ende (106) und einem distalen Ende (108) entlang einer Längsachse erstreckt und eine Vielzahl von Fensterungen aufweist, die eine Vielzahl von sich axial erstreckenden Balken (410) und sich in Umfangsrichtung erstreckenden Ringen (634) in einem Ein-Balken-Schnittmuster definieren, wobei ein einzelner sich axial erstreckender Balken (310) zwischen jedem Paar benachbarter sich in Umfangsrichtung erstreckender Ringe (634) angeordnet ist;
wobei mindestens ein Balken (410) des Ein-Balken-Schnittmusters eine Innenfläche (420), eine Außenfläche (422) und ein Paar Seitenflächen (424) umfasst, **dadurch gekennzeichnet, dass**
ein Winkel (430), der zwischen der Innenfläche (420) und einer oder beiden der Seitenflächen (424a, 424b) gebildet wird, zwischen 80 Grad und 120 Grad beträgt.

2. Vorrichtung gemäß Anspruch 1, wobei das längliche Element (104) eine Rohrstruktur (214) ist.

3. Vorrichtung gemäß Anspruch 2, ferner umfassend mindestens eines von einem Kern (212), der innerhalb der Rohrstruktur (214) angeordnet ist, einem Innenrohr, das innerhalb der Rohrstruktur angeordnet ist, oder einer oder mehreren Spulen (224), die innerhalb der Rohrstruktur (214) angeordnet sind.

4. Vorrichtung gemäß irgend einem der Ansprüche 1 bis 3, wobei die Ringe (634) und Balken (410) in einem Polymer eingekapselt sind.

5. Vorrichtung gemäß irgend einem der Ansprüche 1 bis 4, wobei das längliche Element (104) eine Nickel-Titan-Legierung oder rostfreien Stahl oder ein Polymer umfasst.

6. Vorrichtung gemäß irgend einem der Ansprüche 1 bis 5, wobei das längliche Element (104) aus zwei oder mehr unterschiedlichen Materialien gebildet ist.

7. Vorrichtung gemäß irgend einem der Ansprüche 1 bis 6, wobei der mindestens eine Balken (410) eine im Wesentlichen gleichmäßige Dicke über seine Umfangslänge aufweist.

8. Vorrichtung gemäß irgend einem der Ansprüche 1 bis 7, wobei ein Winkel (431), der zwischen der Außenfläche (422) und einer oder beiden Seitenflächen (424) gebildet wird, größer als etwa 45 Grad ist.

9. Vorrichtung gemäß irgend einem der Ansprüche 1 bis 8, wobei die intravaskuläre Vorrichtung (100) ein Führungsdraht ist.

10. Vorrichtung gemäß irgend einem der Ansprüche 1 bis 8, wobei die intravaskuläre Vorrichtung (100) ein Mikrokatheter ist.

11. Verfahren zum Herstellen einer intravaskulären Vorrichtung (100) gemäß Anspruch 1, wobei das Verfahren umfasst:
Bereitstellen eines Stücks von Ausgangsmaterial (402);
Führen einer Klinge (404) in das Ausgangsmaterial (402), um einen ersten Schnitt in dem Ausgangsmaterial (402) zu bilden, ohne vollständig durch das Ausgangsmaterial (402) hindurchzugehen, wobei die Klinge (404) derart ausgerichtet ist, dass eine Schneidkante im Wesentlichen senkrecht zu einer Längsachse des Ausgangsmaterials (402) ist;
Drehen des Ausgangsmaterials (402) relativ zu der Klinge (404) um etwa 60 Grad bis etwa 120 Grad, ohne das Ausgangsmaterial (402) relativ zu der Klinge (404) in Längsrichtung vorzuschieben; und
Führen der Klinge (404) in das Ausgangsmaterial (402), um einen zweiten Schnitt zu bilden.

12. Verfahren gemäß Anspruch 11, wobei der erste Schnitt durch Führen der Klinge (404) in das Ausgangsmaterial (402) bis zu einer Tiefe von etwa 30 % bis etwa 70 % des Durchmessers des Ausgangsmaterials (402) durchgeführt wird.

13. Verfahren gemäß Anspruch 11 oder Anspruch 12, wobei der zweite Schnitt durch Führen der Klinge (404) in das Ausgangsmaterial (402) bis zu einer Tiefe von etwa 30 % bis etwa 70 % des Durchmessers des Ausgangsmaterials (402) durchgeführt wird.

## Revendications

1. Dispositif intravasculaire (100), comprenant :
un organe allongé (104) s'étendant entre une extrémité proximale (106) et une extrémité distale (108), et une pluralité de fenêtres définissant une pluralité de traverses (410) s'étendant de manière axiale et d'anneaux (634) s'étendant de manière circonférentielle, selon un motif de découpe à un seul faisceau, selon lequel une seule traverse s'étendant de manière axiale (310) est disposée entre chaque paire adjacente d'anneaux s'étendant de manière circonférentielle (634) ;
selon lequel au moins une traverse (410) parmi le motif de découpe à un seul faisceau comporte une surface intérieure (420), une surface extérieure (422), et une paire de surfaces latérales (424),
**caractérisé en ce qu'**un angle (430) formé entre la surface intérieure (420) et une ou les deux surfaces latérales (424a, 424b) se situe entre 80 degrés et 120 degrés.

2. Dispositif selon la revendication 1, selon lequel l'organe allongé (104) se présente sous forme de structure tubulaire (214).

3. Dispositif selon la revendication 2, comprenant en outre au moins un élément parmi une âme (212) disposée à l'intérieur de la structure tubulaire (214), un tube intérieur disposé à l'intérieur de la structure tubulaire, ou une ou plusieurs bobines (224) disposées à l'intérieur de la structure tubulaire (214).

4. Dispositif selon l'une quelconque des revendications 1 à 3, selon lequel les anneaux (634) et les traverses (410) sont enrobés d'un polymère.

5. Dispositif selon l'une quelconque des revendications 1 à 4, selon lequel l'organe allongé (104) comprend un alliage en nickel-titane ou en acier inox ou en polymère.

6. Dispositif selon l'une quelconque des revendications 1 à 5, selon lequel l'organe allongé (104) est formé à partir de deux matériaux différents ou plus.

7. Dispositif selon l'une quelconque des revendications 1 à 6, selon lequel la au moins une traverse (410) présente une épaisseur sensiblement uniforme à travers sa longueur circonférentielle.

8. Dispositif selon l'une quelconque des revendications 1 à 7, selon lequel un angle (431) formé entre la surface extérieure (422) et l'une ou les deux surfaces latérales (424) est supérieur à environ 45 degrés.

9. Dispositif selon l'une quelconque des revendications 1 à 8, selon lequel le dispositif intravasculaire (100) est un fil guide.

10. Dispositif selon l'une quelconque des revendications 1 à 8, selon lequel le dispositif intravasculaire (100) est un micro-cathéter.

11. Procédé de fabrication d'un dispositif intravasculaire (100) selon la revendication 1, le procédé comprenant :
la mise à disposition d'un morceau de matériau de base (402) ;
le passage d'une lame (404) dans le matériau de base (402) afin de former une première découpe dans le matériau de base (402) sans traverser complètement le matériau de base (402), la lame (404) étant orientée de sorte qu'un bord de coupe se trouve sensiblement perpendiculaire à un axe longitudinal du matériau de base (402) ;
la rotation du matériau de base (402) par rapport à la lame (404) de l'ordre d'environ 60 degrés à 120 degrés sans faire avancer longitudinalement le matériau de base (402) par rapport à la lame (404) ; et
le passage de la lame (404) dans le matériau de base (402) afin de former une deuxième découpe.

12. Procédé selon la revendication 11, selon lequel la première découpe est effectuée en passant la lame (404) dans le matériau de base (402) jusqu'à une profondeur d'environ 30% à 70% du diamètre du matériau de base (402).

13. Procédé selon la revendication 11 ou la revendication 12, selon lequel la deuxième découpe est effectuée en passant la lame (404) dans le matériau de base (402) jusqu'à une profondeur d'environ 30 % à 70 % du diamètre du matériau de base.
